# EUROPEAN PATENT APPLICATION

(11) **EP 1 929 994 A1**
(43) Date of publication of application: **11.06.2008**
(21) Application number: 06798285.0
(22) Date of filing: 25.09.2006
(51) Int. Cl.: A61K 8/38, A61Q 11/00

(54) **TEETH WHITENING MATERIAL AND TEETH WHITENING METHOD**

(30) Priority: 28.09.2005 JP 2005281506
(71) Applicant: MITSUBISHI GAS CHEMICAL COMPANY, INC., Chiyoda-ku, Tokyo 100-8324 (JP)
(72) Inventor: KAWABATA, Yasunari, Katsushika-ku Tokyo 125-0051 (JP); HIRAMATSU, Yasushi, Katsushika-ku Tokyo 125-0051 (JP); KURATA, Hiroshi, Katsushika-ku Tokyo 125-0051 (JP)
(74) Representative: Gille Hrabal Struck Neidlein Prop Roos
(86) International application number: PCT/JP2006/318954
(87) International publication number: WO 2007/037197

(57) **Abstract**

The present invention relates to a tooth bleaching material containing peroxyglutaric acid and/or peroxypolyacrylic acid as a bleaching ingredient, as well as a method of bleaching teeth which include the step of applying the tooth bleaching material in the form of a dilute solution or without dilution onto surfaces of discolored teeth. In accordance with the present invention, there are provided a tooth bleaching material for bleaching and removing colored sediments (colored or discolored deposits) on teeth which do not essentially require irradiation with light, and is readily used with a high safety and further suitable as a domestic bleaching material, as well as a tooth bleaching method using the above tooth bleaching material.

## Description

### TECHNICAL FIELD

The present invention relates to tooth bleaching materials for bleaching and removing colored sediments (colored or discolored deposits) on teeth, and methods for bleaching tooth.

### BACKGROUND ART

In recent years, there has been an increasing demand for esthetic improvements of teeth such as improvements of contours, alignment and integrity of teeth in dental therapy. In particular, there have been more cases of young women desiring such a dental therapy so as to whiten teeth as an important element of beauty. In general, the cause of dental discoloration and pigmentation or stain falls into the two categories: extrinsic causes such as sedimentation of colored materials (tobacco, tea, etc.), pigment generating bacteria, discoloration of filling materials (primarily composite resins) and metal salts (primarily amalgam, silver nitrate, and ammonia silver); and intrinsic causes such as aging, chemicals or medicine (fluorine, tetracyclines, etc.), dysmetabolism and hereditary, and dental injuries.

Several methods have been proposed as methods for esthetic improvement of discolored teeth, among which bleaching may be considered as a highly effective method for the preservation of dentine, although there may be some cases of color reversion. Bleaching is basically a method for decoloring colored materials through a chemical reaction. In the past, there were various reports of bleaching materials comprising a variety of chemicals based on vital bleaching and non-vital bleaching as well as bleaching methods using such bleaching materials.

On the other hand, bleaching methods and bleaching materials for dental bleaching require the following conditions:
(a) pronounced bleaching results;
(b) non-toxicity of bleaching materials used;
(c) easy operations;
(d) no detraction to dental physical properties after bleaching;
(e) efficacious for vital tooth bleaching as well as non-vital tooth bleaching; and
(f) speedy bleaching results, and so on.
   However, in conventional bleaching materials or methods, the primary bleaching agent is 30% to 35% aqueous hydrogen peroxide, which is highly corrosive and whose oxidative property is the base for bleaching teeth, resulting in problems in terms of simplicity and safety.

Taking the afore-mentioned status of the bleaching methods into consideration, it has been reported that a beaching material and bleaching method using combination of titanium dioxide having a photocatalytic action and a low concentration aqueous solution of hydrogen peroxide without using highly toxic 30 to 35 wt% aqueous hydrogen peroxide, are effective as new bleaching material and method showing excellent simplicity and safety which create remarkable effects to vital teeth as well as non-vital teeth for a short period of time (refer to Patent Document 1). However, this method inevitably requires irradiation with light, and it may be difficult to use the bleaching material in a domestic bleaching application (take-home bleaching).

Meanwhile, Patent Document 2 discloses an esthetic tooth whitening composition containing glutaric acid. However, the glutaric acid blended in the composition serves for promoting dissolution of a calcium phosphate compound and improving a shaping property of a ground paste. In the Patent Document 2, there is no description concerning peroxyglutaric acid. Also, there have been many reports concerning examples of tooth bleaching materials containing a polyacrylic acid as a thickening agent. However, in these reports, there are no descriptions concerning peroxypolyacrylic acid.
Patent Document 1: Japanese Patent Application Laid-open No. H11-92351/1999
Patent Document 2: Japanese Patent Application Laid-open No. H11-116421/1999

### DISCLOSURE OF THE INVENTION

An object of the present invention is to provide a tooth bleaching material that does not essentially require irradiation with light, and is readily used with a high safety and further suitable as a domestic bleaching material.

As a result of intensive and extensive researches as to the above tooth bleaching material, the present inventors have found that the tooth bleaching material containing peroxyglutaric acid and/or peroxypolyacrylic acid as a bleaching ingredient is excellent in bleaching effect as well as safety even without irradiation with light. The present invention has been accomplished on the basis of the finding. That is, the present invention relates to a tooth bleaching material comprising peroxyglutaric acid and/or peroxypolyacrylic acid as a bleaching ingredient.

### BEST MODE FOR CARRYING OUT THE INVENTION

The present invention is described in more detail below. The present invention is characterized by a tooth bleaching material comprising peroxyglutaric acid and/or peroxypolyacrylic acid as a bleaching ingredient.

The peroxyglutaric acid is produced by reacting glutaric acid or glutaric anhydride with hydrogen peroxide, and obtained in the form of an aqueous solution containing peroxyglutaric acid, hydrogen peroxide and glutaric acid. The peroxypolyacrylic acid is produced by reacting polyacrylic acid with hydrogen peroxide under an acidic condition, and obtained in the form of an aqueous solution containing peroxypolyacrylic acid as a modified polyacrylic acid formed by converting a part of carboxyl groups of the polyacrylic acid into a peroxycarboxyl group, and hydrogen peroxide. The peroxyglutaric acid or peroxypolyacrylic acid may be produced in the presence of an acid catalyst or a stabilizer. Examples of the acid catalyst include sulfuric acid, orthophosphoric acid and pyrophosphoric acid. As the stabilizer, there may be used any suitable compound as long as it is known as a stabilizer for peracids. Examples of the stabilizer include orthophosphoric acid, 1-hydroxyethylene-1,1-diphosphonic acid, pyrophosphoric acid, sodium hexametaphosphate and dipicolinic acid.

Next, the method of calculating a concentration of each of the peroxyglutaric acid and the peroxypolyacrylic acid is explained. A potassium permanganate solution is added to a solution of the peroxyglutaric acid or the peroxypolyacrylic acid until a pink color of the potassium permanganate disappears to thereby decompose hydrogen peroxide contained in the solution of the peroxyglutaric acid or the peroxypolyacrylic acid. Successively, the concentration of a peroxycarboxyl group in the resultant solution is measured by iodimetry. The concentration of the peroxyglutaric acid is calculated based on a monoperoxyglutaric acid (HOOCCH₂CH₂CH₂COOOH; molecular weight: 148), whereas the concentration of the peroxypolyacrylic acid is calculated based on a polymer constituting unit thereof (-CH₂CHCOOOH-; molecular weight: 88).

The concentration of the peroxyglutaric acid or the peroxypolyacrylic acid in the tooth bleaching material of the present invention is not particularly limited as long as a sufficient tooth bleaching effect can be attained. However, from the viewpoints of a good bleaching effect and a low irritativeness, the concentration of the peroxyglutaric acid or the peroxypolyacrylic acid in the tooth bleaching material is preferably from 0.5 to 35% by weight. That is, when the concentration of the peroxyglutaric acid or the peroxypolyacrylic acid in the tooth bleaching material is 0.5% by weight or more, a sufficient bleaching effect can be attained, whereas when the concentration of the peroxyglutaric acid or the peroxypolyacrylic acid in the tooth bleaching material is 35% by weight or less, adverse influence on human body such as irritation to skin can be suppressed. From these viewpoints, the concentration of the peroxyglutaric acid or the peroxypolyacrylic acid in the tooth bleaching material is more preferably from 0.5 to 10% by weight.

The tooth bleaching material of the present invention may also contain a thickening agent in order to facilitate application of the bleaching material onto the surfaces of teeth. The thickening agent may be previously mixed in the above bleaching ingredient or may be mixed in the bleaching material immediately before used. The thickening agent may be either organic or inorganic. The inorganic thickening agent used in the bleaching material is preferably an inorganic clay mineral and more preferably a layered structure type inorganic clay mineral. In general, the inorganic clay minerals are classified into fibrous structure type (for example, sepiolite, attapulgite, etc.), an amorphous structure type (for example, allophane, etc.), a mixed layer structure type (for example, kaolinite, montmorillonite, etc.) and the above layered structure type. The layered structure type inorganic clay minerals take water molecules into a unit space between the layers and then swelled. By utilizing this property, the peroxide contained in the bleaching material is to be held as adhered onto the surfaces of the discolored teeth. In the present invention, the layered structure type inorganic clay minerals capable of being swelled in the presence of water are preferably used.

Although the inorganic clay minerals of fibrous structure type and amorphous structure type are also possible to be swelled by adding water and then mixing therewith using a high speed mixer, the layered structure type clay minerals are more advantageous because no such special apparatus is required.

Examples of the inorganic clay minerals include dickite, nacrite, kaolinite, anorthite, halloysite, metahalloysite, chrysotile, lizardite, serpentine, antigorite, beidellite, montmorillonite, sauconite, stevensite, nontronite, saponite, hectorite, vermiculite, smectite, sepiolite, illite, sericite, glauconite-montmorillonite, roselite-montmorillonite, chlorite-vermiculite, illite-montmorillonite, halloysite-montmorillonite and kaolinite-montmorillonite.

Among the above inorganic clay minerals, montmorillonite, sauconite, smectite, stevensite, beidellite, nontronite, saponite, hectorite, vermiculite, nacrite and sepiolite are especially preferably used as the layered structure type clay minerals in the present invention. These inorganic clay minerals may be either natural products or synthetic products. Examples of the synthetic inorganic clay minerals include synthesized magnesium sodium lithium silicate (laponite). These inorganic clay minerals may also be used in the form of a mixture of any two or more thereof.

Examples of the organic thickening agent include sodium alginate, propylene glycol alginate, sodium carboxymethyl cellulose, sodium starch glycolate, sodium starch phosphate, methyl cellulose, sodium polyacrylate, polyacrylic acid, acrylic acid/maleic acid copolymers, polyoxyalkylene alkyl ethers, glycerol, ethylene glycol, diethylene glycol, polyethylene glycol, propylene glycol and polypropylene glycol. These organic thickening agents may be either natural products or synthetic products, and may also be used in the form of a mixture of any two or more thereof.

The amount of the thickening agent blended is not particularly limited as long as it is selected such that the tooth bleaching material of the present invention has a desired viscosity, and is preferably from 0.01 to 20% by weight and more preferably from 0.1 to 5% by weight on the basis of a total amount of the tooth bleaching material. The viscosity of the tooth bleaching material is preferably from 0.001 to 30 Pa·s. The tooth bleaching material having a viscosity of the above specified range is free from sagging even when applied to the tooth surface inclined at an angle of 45° relative to a horizontal plane. From the above viewpoints, the viscosity of the tooth bleaching material is more preferably from 0.01 to 10 Pa·s.

The solution of the peroxyglutaric acid or the peroxypolyacrylic acid usually exhibits a strong acidity from the viewpoint of a good preservation stability thereof. Therefore, if the solution is directly applied to the surfaces of the teeth, there arises such a risk that the teeth is severely injured. From the viewpoint of avoiding occurrence of the injured teeth, it is preferred that a pH controller is added to the tooth bleaching material of the present invention upon use to adjust a pH thereof to from 3 to 8. Examples of the pH controller include aqueous solutions of inorganic alkalis such as sodium hydroxide, potassium hydroxide, sodium phosphate, sodium hydrogenphosphate, potassium phosphate, potassium hydrogenphosphate, sodium carbonate and sodium hydrogencarbonate. The pH of the tooth bleaching material is more preferably from 5 to 8.

The peroxyglutaric acid or the peroxypolyacrylic acid is preferably in the form of a high-concentration solution from the viewpoint of a good production efficiency. Therefore, the tooth bleaching material of the present invention may be suitably diluted upon use such that the concentration of the bleaching ingredient therein is adjusted to a necessary level. The diluent may be water, and may also contain the above thickening agent, pH controller, etc. In addition, the diluent may also contain a perfume to attain a good feeling upon use.

As described above, the tooth bleaching material of the present invention is applied to the surfaces of the discolored teeth in the form of a dilute solution or without dilution to thereby exhibit a sufficient bleaching effect without irradiation with light. However, the tooth bleaching material can be further enhanced in bleaching effect by irradiating light thereto. In the case of irradiating the bleaching material with light, the tooth bleaching material is directly applied to the surfaces of the teeth to be treated, and light is irradiated to the surfaces one or more times and preferably repeatedly plural times. The light to be irradiated preferably has a wavelength giving a less adverse influence on human body. The wavelength of light to be irradiated is preferably 300 nm or more and more preferably from 380 to 500 nm. Examples of a light source used for the light irradiation include an incandescent lamp, a fluorescent lamp, a halogen light bulb, a black light, a metal halide lamp, a xenon lamp, a mercury lamp, a UV lamp, a LED (light emitted diode) lamp and a semiconductor laser. The light from these light sources may be passed through an adequate filter to cut out unnecessary wavelengths therefrom, thereby attaining light having a desired wavelength.

The application of the bleaching material as well as the irradiation of light thereto may be repeated optional times according to the degree of the tooth discoloration. Upon conducting the application procedure, the bleaching material may be usually applied at intervals of from about 15 to about 20 min, and the interval and frequency of the application of the bleaching material may be appropriately determined according to the conditions of the teeth. The bleaching material of the present invention is effective to bleach both vital teeth and non-vital teeth.

### EXAMPLES

The present invention is described in more detail below by referring to the following examples. However, the following examples are only illustrative, and not intended to limit the invention thereto.

### EXAMPLE 1

A mixture of 2.5 g of glutaric acid, 4 mL of a 35 wt% hydrogen peroxide aqueous solution, 0.1 mL of 95 wt% sulfuric acid and 0.01 g of dipicolinic acid was dissolved and allowed to stand at room temperature for 3 days, thereby obtaining a tooth bleaching material containing 26% by weight of peroxyglutaric acid and 15% by weight of hydrogen peroxide.
Further, the thus obtained tooth bleaching material was diluted with purified water, and a pH of the material was suitably adjusted by adding sodium hydroxide thereto, thereby obtaining a tooth bleaching material containing 3.7% by weight of peroxyglutaric acid and 2.1% by weight of hydrogen peroxide and having a pH of 5.3 (amount of active oxygen: 1.4%).

### EXAMPLE 2

A mixture of 2 g of polyacrylic acid, 6 mL of a 35 wt% hydrogen peroxide aqueous solution, 0.1 mL of 95 wt% sulfuric acid and 0.01 g of dipicolinic acid was dissolved and allowed to stand at room temperature for 14 days, thereby obtaining a tooth bleaching material containing 6.5% by weight of peroxypolyacrylic acid and 23% by weight of hydrogen peroxide.
Further, the thus obtained tooth bleaching material was diluted with purified water, and a pH of the material was suitably adjusted by adding sodium hydroxide thereto, thereby obtaining a tooth bleaching material containing 0.8% by weight of peroxypolyacrylic acid and 2.7% by weight of hydrogen peroxide and having a pH of 5.5 (amount of active oxygen: 1.4%).

### COMPARATIVE EXAMPLE 1

A 3 wt% hydrogen peroxide aqueous solution whose pH was adjusted to 5.5 (amount of active oxygen: 1.4%) was prepared.

### COMPARATIVE EXAMPLE 2

An aqueous solution containing 5% by weight of glutaric acid and 3% by weight of hydrogen peroxide and having a pH of 5.3 (amount of active oxygen: 1.4%) was prepared.

### COMPARATIVE EXAMPLE 3

An aqueous solution containing 2.6% by weight of polyacrylic acid and 3% by weight of hydrogen peroxide and having a pH of 5.3 (amount of active oxygen: 1.4%) was prepared.

### COMPARATIVE EXAMPLE 4

A 30 wt% hydrogen peroxide aqueous solution whose pH was adjusted to 3.1 (amount of active oxygen: 14%) was prepared. The thus prepared hydrogen peroxide aqueous solution exhibited a strong irritativeness to skin.

### EXAMPLE 3

The same procedure as in Example 1 was repeated except that a thickening agent (sodium polyacrylate) was added upon diluting with purified water, thereby obtaining a tooth bleaching material containing 3.7% by weight of peroxyglutaric acid, 2.1% by weight of hydrogen peroxide and 1.0% by weight of the thickening agent and having a pH of 5.3 (amount of active oxygen: 1.4%). The thus obtained tooth bleaching material was in the form of a high-viscous composition and readily applied to the surfaces of teeth.

### EXAMPLE 4

The same procedure as in Example 2 was repeated except that a thickening agent (sodium polyacrylate) was added upon diluting with purified water, thereby obtaining a tooth bleaching material containing 0.8% by weight of peroxypolyacrylic acid, 2.7% by weight of hydrogen peroxide and 1.0% by weight of the thickening agent and having a pH of 5.5 (amount of active oxygen: 1.4%). The thus obtained tooth bleaching material was in the form of a high-viscous composition and readily applied to the surfaces of teeth.

To examine a bleaching effect of the tooth bleaching material according to the present invention, the bleaching material was subjected to (1) a methylene blue bleaching test and (2) a bleaching test for discolored teeth.

### (1) Methylene Blue Bleaching Test

A model bleaching test using no irradiation with light was conducted as follows. A testing solution prepared by dissolving 10 ppm of methylene blue (blue dye) in the tooth bleaching material was filled in a quartz cell of 1 cm cube and allowed to stand in a dark place for 30 min to measure a decomposition rate of methylene blue in the solution.
In addition, a model bleaching test using irradiation with light was conducted as follows. A testing solution prepared by dissolving 10 ppm of methylene blue (blue dye) in the tooth bleaching material was filled in a quartz cell of 1 cm cube and irradiated with light for 5 min using a dental visible light irradiator (tradename " OPTILUX 501" available from Demetron Inc.) to measure a decomposition rate of methylene blue in the solution. The results of the above methylene blue bleaching test for the tooth bleaching materials obtained in Examples 1 and 2 and Comparative Examples 1 to 4 are shown in Table 1.

### (2) Bleaching Test for Discolored Teeth

By using the tooth bleaching materials obtained above, discolored teeth (extracted teeth) were subjected to a bleaching test by the following procedure.
1) As preliminary arrangements, plaque, tartar, tar, etc., were removed from the discolored teeth using an ultrasonic wave scaler.
2) The surfaces of the teeth were cleaned by an ordinary method using a rubber-cup, etc., and then dried.
3) A plain moisture-proofing method was carried out.
4) The tooth bleaching material was applied to the surfaces of the teeth, followed by irradiating light to the surfaces using a dental visible light irradiator (tradename " OPTILUX 501" available from Demetron Inc.).
5) Light was irradiated for 2 min every time. Upon each test, a fresh tooth bleaching material was applied and irradiated with light for the above period of time. This procedure was repeated 10 times (total irradiation time: 20 min).
   The color of the surfaces of the teeth after subjected to the bleaching treatment was measured by a spectrophotometer ("SE-2000" available from Nippon Denshoku Co., Ltd.) to represent the change in color of the discolored teeth by L* (lightness), a* (redness) and b* (yellowness). The results of the above bleaching test for the tooth bleaching materials obtained in Examples 1 and 2 and Comparative Examples 1 to 4 are shown in Table 2.

**TABLE 1: Results of Methylene Blue Bleaching Test**

| | Rate of decomposition of methylene blue | |
|---|---|---|
| | After placed in dark place for 30 min | After irradiated with light for 5 min |
| Example 1 | 84% | 63% |
| Example 2 | 36% | 26% |
| Comparative Example 1 | 0% | 5% |
| Comparative Example 2 | 0% | 7% |
| Comparative Example 3 | 0% | 6% |
| Comparative Example 4 | 2% | 9% |

**TABLE 2: Result of Bleaching Test for Discolored Teeth**

| | Color difference values | | | | | |
|---|---|---|---|---|---|---|
| | Before bleaching treatment | | | After irradiated with light for 20 min | | |
| | L* | a* | b* | L* | a* | b* |
| Example 1 | 59 | -3 | 2 | 63 | -2 | -1 |
| Example 2 | 65 | -2 | 5 | 68 | -2 | 5 |
| Comparative Example 1 | 58 | -3 | -1 | 58 | -3 | -1 |
| Comparative Example 2 | 60 | -2 | -1 | 60 | -2 | -1 |
| Comparative Example 3 | 63 | -3 | -2 | 63 | -3 | -2 |
| Comparative Example 4 | 60 | -2 | -1 | 63 | -2 | -1 |

### INDUSTRIAL APPLICABILITY

The present invention relates to a tooth bleaching material containing peroxyglutaric acid and/or peroxypolyacrylic acid as a bleaching ingredient, as well as a method of bleaching teeth which include the step of applying the tooth bleaching material in the form of a dilute solution or without dilution onto surfaces of discolored teeth. In accordance with the present invention, there are provided a tooth bleaching material for bleaching and removing colored sediments (colored or discolored deposits) on teeth which do not essentially require irradiation with light, and is readily used with a high safety at low costs and further suitable as a domestic bleaching material, as well as a tooth bleaching method using the above material.

## Claims

1. A tooth bleaching material comprising peroxyglutaric acid and/or peroxypolyacrylic acid as a bleaching ingredient.

2. The tooth bleaching material according to claim 1, wherein the bleaching ingredient is contained in an amount of from 0. 5 to 35% by weight.

3. The tooth bleaching material according to claim 1 or 2, further comprising a thickening agent.

4. The tooth bleaching material according to any one of claims 1 to 3, wherein a pH of the tooth bleaching material is controlled to from 3 to 8 upon use.

5. A method of bleaching teeth, comprising the step of applying the tooth bleaching material as defined in any one of claims 1 to 4 in the form of a dilute solution or without dilution, onto surfaces of discolored teeth.

6. The method according to claim 5, further comprising the step of irradiating light to the surfaces of the discolored teeth onto which the tooth bleaching material is applied.
